# EUROPEAN PATENT SPECIFICATION

(11) **EP 1 539 684 B1**
(45) Date of publication and mention of the grant of the patent: **15.04.2009**
(21) Application number: 03793925.3
(22) Date of filing: 08.09.2003
(51) Int. Cl.: C07C 303/40, C07C 311/37, A61K 31/18

(54) **BENZENESULPHONAMIDES AND PROCESS FOR THEIR PREPARATION**
BENZOLSULFONAMIDE UND VERFAHREN ZU IHRER HERSTELLUNG
BENZENESULPHONAMIDES ET PROCEDES D'ELABORATION CORRESPONDANTS

(30) Priority: 09.09.2002 HU 0202963
(43) Date of publication of application: 15.06.2005
(73) Proprietor: Richter Gedeon Nyrt., 1103 Budapest (HU)
(72) Inventor: GIZUR, Tibor, H-1162 Budapest (HU); TÖRLEY, Jozsef, H-1137 Budapest (HU); FOGASSY, Elemér, H-1112 Budapest (HU); BALINT, Jozsef, H-1131 Budapest (HU); EGRI, Gabriella, H-1131 Budapest (HU); DEMETER, Adám, H-1106 Budapest (HU)
(86) International application number: PCT/HU2003/000071
(87) International publication number: WO 2004/022532

(56) References cited:
- EP-A- 0 257 787
- EP-A- 1 057 813
- GB-A- 1 123 565
- US-A- 3 860 647
- DATABASE CAPLUS AMERICAN CHEMICAL SOCIETY; 2000, XP002264998 retrieved from STN Database accession no. 2000:585409 -& CHEMICAL ABSTRACTS, vol. 133, no. 13, 25 September 2000 (2000-09-25), Columbus, Ohio, US; abstract no.: 177015, XP002264997 & JP 2000 029901 A (YAMANOUCHI PHARMACEUTICAL CO LTD) 22 August 2000

## Description

The invention relates to new benzenesulphonamides and process for the preparation of a known benzenesulphonamide derivative of formula I.

This benzenesulphonamide derivative of formula I - known as R (-)-tamsulosine HCI, the chemical name: R(-)-5-[2-[[2-(2-ethoxy-phenoxy)-ethyl]-amino]-propyl-2-methoxy-benzenesulphonamide hydrochloride] - is the active ingredient of a drug for the treatment of Benign Prostatic Hyperplasia (BPH).

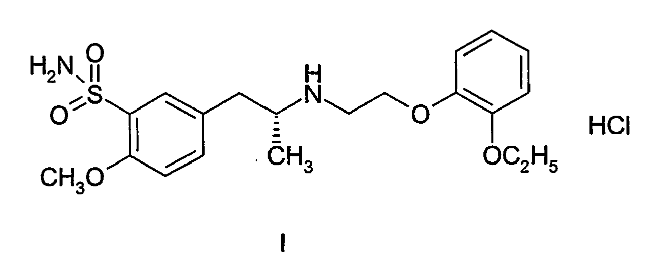

There are several processes for the preparation of the racemic or optically active form of the compound of formula I.

EP 34432 describes a process for the preparation of the racemic form of the compound of formula I. According to this process the product of formula I can be produced from substituted phenyl acetone and 2-(2-amino-ethoxy)-phenetol. The condensed derivative of the starting materials is reduced to the desired racemic product with hydrogen in the presence of platinum oxide catalyst. According to another synthetic method the hydroxyl group is replaced to chlorine by thionyl chloride and the racemic product is obtained by reduction in the presence of palladium catalyst. The disadvantage of the process is that not the optically active product is formed.

JP 62 119 952 describes the preparation of R-(-)-tamsulosine HCl, where R-(-)-2-(4-methoxy-phenyl)-1-methylamine is used as starting material. The amino group is protected by acetylation, thereafter the aromatic ring chlorosulphonated and sulphamoyl group is formed by reaction of the obtained product with ammonia. This compound is reacted with 2-(2-ethoxy-phenoxy)-acetaldehyde dietylacetal in the presence of platinum oxide catalyst, and after removing the acetyl group R-(-)-tamsulosine HCl is obtained. The yields are not known. The disadvantages of the process are that the used reagents are very costly, the synthesis is too complicated and industrially not applicable.

EP 257 787 describes R-(-)-5-(2-amino-propyl)-2-methoxy-benzenesulphonamide HCl as starting material. According to JP 18 353 this compound can be prepared only by a very complicated method. The base form of this compound is reacted with 2-(2-ethoxy-phenoxy)-ethyl-bromide, the above base is used as acid neurtalizing agent too. The yield is very low, only 30%, the product is purified by column chromatography. The disadvantages of the process are the costly starting material, very low yield calculating on the optically pure starting amine (this is used as acid neurtalizing agent too) and the purification by column chromatography.

EP 380 144 describes a similar process as above, according to this R-(-)-5-(2-aminopropyl)-2-methoxy-benzenesulphonamide is prepared by reacting (4-methoxy-3-sulphamoyl-phenyl)-acetone with R-(-)-α -methyl-benzylamine. The reductive condensation is performed in methanol in the presence of platinum oxide or Raney nickel catalyst under hydrogen stream. The obtained diastereomer mixture is recrystallized four times from acetone or acetonitrile to give the required optical purity. The obtained optically pure product is reduced in methanol in the presence of palladium carbon catalyst to provide the optically pure amine. The preparation of the end-product is described in EP 257 787. The disadvantages of the process are the costly reagents [R-(-)-α-methyl-benzyl-amine, platinum oxide] and the low yield calculated on the above costly amine (12%).

In summary: according to the processes of the prior art the compound of R-(-)-tamsulosine HCl of formula I can be synthesised only in a very low yield, in several steps and by using very costly starting materials or the processes did not provide the optically pure product.

The object of the invention is to elaborate a production process, which eliminates the disadvantages of the known processes and according to which the compound of R-(-)-tamsulosine HCl of formula I can be synthesised in optically pure form, using simple technology, cheap starting materials and from this high quality active ingredient stabile pharmaceutical composition can be produced.

The recognition according to our invention is that the best starting material for the preparation of compound of formula I is the secondary amine of formula II, the chemical name is (R)-(-)-2-methoxy-5-(2-benzylamino-propyl) benzoyl-sulphonamide, which is a new compound:
The compound of formula II can be prepared easily by reductive condensation of the known (4-methoxy-3-sulphamoyl-phenyl)-acetone and the cheap benzylamine.

Surprisingly it was found that the costly hydrogenation catalysts are not needed, the reaction is performed in good yield in the presence of sodium borohydride too.

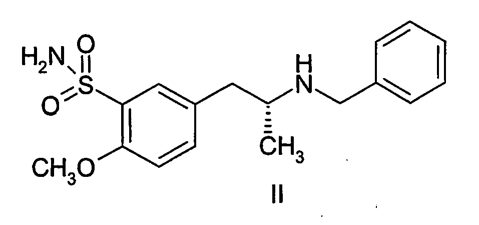

Also the recognition according to our invention is that the racemic form of the obtained compound of formula II can be optically resolved with the easily available natural tartaric acid or its benzoyl derivative.

Further recognition according to our invention is that the optically pure secondary amine of formula II obtained by the above optical resolution is more applicable for the alkylation reaction than R-(-)-5-(2-amino-propyl)-2-methoxy-benzenesulphonamide, prepared by debenzylation in the patents of the prior art, because with secondary amine in the alkylation reaction the dialkylation can be avoided.

Surprisingly it was found that (2-mesyloxy-ethoxy)-phenetol of formula IV is a more applicable alkylating agent then the bromine derivative described in the prior art processes.

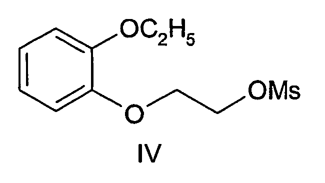

Further recognition according to our invention is that in the alkylating reaction magnesium oxide can be used as naturalize agent and so the need of two equivalents amine can be avoided. This is surprising, because the sulphamoyl group is strongly acidic and reacts with bases salt forming. This sulphamoyl group reacts rather with alkylating agent and N,N-disubstituted sulphamoyl compounds are produced.

It was found by our experiments that magnesium oxide - as an acid - is suitable to promote the reaction in an organic solvent, because the reaction of sulphamoyl group can be avoided. Therefore the use of secondary amine in an equivalent amount is enough.

The tertiary amine of formula III, chemical name: R-5-[2-[N-(2-ethoxy-phenoxy)-ethyl]-N-benzyl]-amino]-propyl-2-methoxy-benzenesulphonamide is prepared by treating the starting material of formula II with compound of formula IV, which is a new compound. From the tertiary amine of formula III the benzyl group can be removed by hydrogenation in the presence of palladium carbon catalyst, thereafter salt is formed to give the optically pure R-(-)-tamsulosine HCl of formula I.

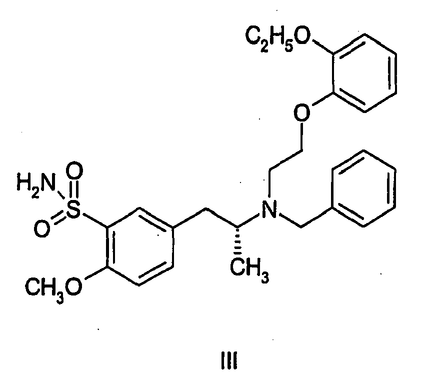

According to the above the subject matter of the invention is the (R,S)-5-[2-(N-benzylamino)-propyl]-2-methoxy-benzene-sulphonamide of formula II, the acid addition salts and enantiomers thereof, furthermore the R-5-[2-[N-(2-ethoxy-phenoxy)-ethyl]-N-benzyl]-amino]-propyl-2-methoxy-benzenesulphonamide, compounds which are the intermediates of the process for the preparation of R-(-)-tamsulosine HCl of formula I.

Further object of the invention is the elaboration of a new, industrially applicable process for the preparation of R-(-)-tamsulosine HCl of formula I by reacting the secondary benzylamine of formula II with (2-mesyloxy-ethoxy)-phenetol (1-2 mol equivalents) as alkylating agent to give the tertiary amine of formula III. Thereafter the benzyl protecting group is removed and the desired salt is formed to provide the product of formula I.

The optically pure secondary benzylamine of formula II is prepared by reductive condensation of (4-methoxy-3-sulphamoyl-phenyl)-acetone and benzylamine followed by optical resolution of the obtained racemic compound with a chiral acid.

Preferably the reductive condensation is performed with 0.5-1.5 mol, more preferably with 1 mol equivalent sodium borohydride in a C₁₋₄ chain alcohol, preferably refluxing in methanol. After removing the alcohol the product is isolated with an organic solvent, preferably with methyl-isobuthyl-ketone and then with ethanol hydrochloric acid salt is formed.

For the optical resolution natural tartaric acid or a benzoyl derivative thereof, preferably dibenzoyl-d-tartaric acid is used.

Most preferably the racemic hydrochloride salt of compound II is suspended in methanol and 0.48 mol equivalent dibenzoyl-d-tartaric acid sodium salt solution in water is added thereto. After cooling the solution is stirred for 48 hours and the filtered salt is recrystallized from the mixture of methanol:water 2:1. The free base is liberated from the salt by cc. ammonium hydroxide and extracted with an organic solvent, preferably with ethyl acetate.

For the optical resolution preferably the mixture of water - C₁₋₄ chain alcohol (0.4-1.0 mol) is used. The obtained salt is recrystallized from the mixture of water - C₁₋₄ chain alcohol.

Preferably magnesium oxide is used as acid naturalize agent. Preferably the reaction is performed by refluxing in an organic solvent in the presence of 1-2 mol equivalents of magnesium oxide.

Most preferably the starting amine of formula II is reacted by changing the solvent, without isolation in an organic solvent, preferably by refluxing in xilol with 1.5 mol equivalent of magnesium oxide and 2-ethoxy-(2-mesyloxy-ethoxy)-benzene of formula IV.

The reduction for removing the benzyl protecting group is performed in a C₁₋₄ chain alcohol, preferably in methanol in the presence of palladium carbon catalyst.

Most preferably the product of formula III obtained by alkylation is purified by clarification; the solvent is changed and hydrogenised without isolation in the presence of palladium carbon in methanol, under atmospheric pressure. After removing the catalyst and the solvent the obtained product of formula I is recrystallized from an organic solvent, preferably from ethyl acetate and in an alcohol with aqueous hydrochloric acid the hydrochloride salt is formed to give the product of formula I. The yield is 63.8% calculated on the secondary amine of formula II, the optical purity thereof is 100%.

The advantages of the process according to our invention can be summarized as follows: in a simple chemical reaction cheap, easily available starting materials are used, the use of complicated apparatus can be avoided and the desired active ingredient of formula I is obtained in high yield with high optical purity, from which stable pharmaceutical composition can be produced.

The invention is illustrated by the following non limiting Examples:

### Example 1

### Synthesis of (-)-5-[2-(N-benzyl-amino)-propyl]-2-methoxy-benzenesulphonamide of formula II

In a 500 ml flask 18.5 g (0.05 mol) of (±)-5-[2-(N-benzyl-amino)-propyl]-2-methoxy-benzenesulphonamide hydrochloride (the racemic form of II) - prepared according to the example 3 - and 210 ml of methanol are added. The obtained suspension is warmed with stirring to 70 °C. In another flask 9 g (0.024 mol) of dibenzoyl-d-tartaric acid monohydrate and 1.26 g (0.012 mol) of sodium carbonate are solved in 90 ml of water at 70 °C and after dissolution is poured to the methanol solution. The obtained suspension is dissolved quickly then allowed to cool to room temperature. The crystallization is started at 25 °C and stirred at this temperature for 48 hours and for 1 hours at 5-10 °C. The diastereomer salt is filtered and recrystallized from the mixture of 120 ml methanol-water 2:1. The hot solution is cooled to 5-10 °C and stirred for an hour at this temperature. The precipitated crystals are filtered and washed with 10 ml of water, to give 16.8 g of the wet product.
The optical activity of the dry product is [α]_{D}²⁰= -70° (C=2 methanol).
The above wet product is suspended in 80 ml of water and then 80 ml of ethyl acetate and 7 ml of cc. ammonium hydroxide are added thereto and stirred for dissolution. The phases are separated and the aqueous phase is extracted with 20 ml of ethyl acetate. The combined organic phases are dried over sodium sulphate, filtered and the solution is evaporated under reduced pressure. The residue is crystallized form isopropanol, filtered, dried to give 5.4 g (65% yield calculated on R enantiomer) of the title compound.
The melting point is 116-119 °C, [α]_{D}²⁰= - 21,4° (c=2 methanol)

### Example 2

### Synthesis of R-5-[2-[N-(2-ethoxy-phenoxy)-ethyl]-N-benzyl]-amino]-propyl-2-methoxy-benzenesulphonamide of formula III

In a 100 ml flask 11.2 g (0.03 mol) of (±)-5-[2-(N-benzyl-amino)-propyl]-2-methoxy-benzenesulphonamide hydrochloride (the racemic form of II) and 120 ml of methanol are added and the suspension is warmed to 70°C. In another flask 5.4 g (0.0144 mol) of R,R-dibenzoyl-tartaric acid monohydrate and 1.59 g (0.03 mol) of sodium carbonate are solved in 60 ml of water at 70°C and after dissolution is poured to the methanol solution. The reaction mixture is cooled to room temperature and stirred for 48 hours at this temperature and for 1 hour at 5-10 °C. The precipitated salt is filtered and recrystallized from the mixture of 70 ml of metanol-water 2:1. The product is filtered, suspended in 60 ml of water, 60 ml of ethyl acetate and then 5 ml of cc. ammonium hydroxide are added. The reaction mixture is stirred for dissolution, the phases are separated, the organic phase is dried and evaporated under reduced pressure. The residue is suspended in 30 ml of xilol, then 3.7 g (0.014 mol) of 2-ethoxy-O-(mesyl-oxy-ethyl)-phenol and 0.58 g (0.014 mol) of magnesium-oxide are added. The mixture is refluxed for 40 hours with intensive stirring. The reaction mixture is cooled, filtered and the filtrate is evaporated under reduced pressure. The residue is purified by column chromatography and crystallized from n-hexane, filtered and dried to give 3.0 g (40 % yield calculated on R enantiomer) of the title compound.

| | |
|---|---|
| Melting point is 65-68 °C | [α]_{D}²⁰= -16,7° (c=1 methanol) |

### Example 3

### Synthesis of (±)-5-[2-(N-benzyl-amino)-propyl]-2-methoxy-benzenesulphonamide hydrochloride (the racemic form of II)

In a 500 ml flask18 g (0.074 mol) of (4-methoxy-3-sulphamoyl-phenyl)-acetone, 200 ml of methanol and 7.92 g (0.074 mol) of benzylamine are added. The mixture is refluxed for 2 hours, cooled to 35-40 °C and the mixture of 2.74 g (0.074 mol) of sodium-borohydride in 30 ml methanol and 1 ml 40% sodium hydroxide is added dropwise. The reaction mixture is stirred for 2 hours at this temperature. After the reaction is over 100 ml of water is added and the methanol solvent is evaporated under reduced pressure. The residue aqueous solution is extracted with 1 x 40 ml and 2 x 25 ml of methyl-isobuthyl-ketone. The combined organic phases are dried over sodium sulphate. The filtrate is acidified with ethanolic hydrochloric acid to pH 1 (approximately 10 ml). The precipitated crystal suspension is stirred at 8-10 °C for 30 minutes, filtered and washed with methyl-isobuthyl-ketone and dried on 80°C to give 15.12 g (yield: 55.2%) of the title compound.

### Example 4

### Synthesis of R-(-)-tamsulosine HCl of formula I

In a 500 ml flask 18.5 g (0.05 mol) of (±)-5-[2-(N-benzyl-amino)-propyl]-2-methoxy-benzenesulphonamide hydrochloride (the racemic form of II) is added and the obtained suspension is warmed to 70°C. In another flask 9 g (0.024 mol) of dibenzoyl-d-tartaric acid monohydrate and 1.26 g (0.012 mol) of sodium carbonate are solved in 90 ml of water at 70 °C, and after dissolution is poured to the methanol solution. The obtained suspension is dissolved quickly and then allowed to cool to room temperature. The crystallization is started at 25 °C, stirred at this temperature for 48 hours and for 1 hours at 5-10 °C. The diastereomer salt is filtered, recrystallized from the mixture of 120 ml of methanol-water 2:1. The hot solution is cooled to 5-10 °C and stirred for an hour at this temperature. The precipitated crystals are filtered and washed with 10 ml of water, to give 16.8 g of wet product.
The optical activity of the dry product is [α]_{D} ²⁰= -70° (C=2 methanol).
The wet product is suspended in 80 ml of water, then 80 ml of ethyl acetate and 7 ml cc. ammonium hydroxide is added and stirred for dissolution. The phases are separated and the aqueous phase is extracted with 20 ml of ethyl acetate. The combined organic phases are dried over sodium sulphate, filtered and the solution is evaporated under reduced pressure. The residue is suspended in xilol to give 5.5 g (65.9% yield, in the form of xilol suspension) of the title compound.

To the above suspension (0.016 mol compound of formula II in the base form) in a 250 ml flask 6.24 g (0.024 mol) of 2-ethoxy-O-(mesyl-oxy-ethyl)-phenol and 0.97 g (0.024 mol) of magnesium-oxide are added and the mixture is refluxed for 37 hours with intensive stirring. The reaction mixture is cooled to 20°C, the magnesium salts are filtered and washed with 2 x 5 ml of xilol. The combined xilol solution is washed with 2 x 10 ml 1% aqueous acetic acid and then 80 ml of methanol is added. The mixture is cooled below 10°C and 80 ml of aqueous hydrochloric acid solution (74.5 ml of water and 5.5 ml of 10% hydrochloric acid) is added to adjust the pH 2-3. The phases are separated and the aqueous phase is washed with 10 ml of xilol. The aqueous phase is stirred with activated carbon at 20-25 °C for ½ hour then filtered and washed with the mixture of 10 ml of methanol-water 1:1. Methanol is evaporated under reduced pressure and to the aqueous residue 75 ml of ethyl acetate is added. The solution is alkalified with 10% sodium hydroxide solution to pH 10 below 15 °C. The phases are separated and the aqueous phase is extracted with 75 ml of ethyl acetate. The combined organic phases are washed with 40 ml of saturated sodium chloride solution and then with 40 ml of water. The solution is dried over sodium sulphate below 15 °C and filtered. To the 7.8 g of residue 230 ml of methanol is added and hydrogenised in the presence of 1.4 g of palladium carbon. The catalyst is filtered, washed with 3 x 10 ml of methanol and the filtrate is evaporated under reduced pressure. The residue is recrystallized from 80 ml of ethyl acetate with 0.25 g of activated carbon. The solution is cooled to 0-5 °C and stirred for 1 hour. The precipitated crystals are filtered, washed with 5 ml of ethyl acetate and then recrystallized again from 70 ml of ethyl acetate without activated carbon. The solution is cooled to 0-5 °C and stirred for 1 hour. The crystals are filtered, washed with 5 ml of ethyl acetate and dried.
The above residue is solved in 46 ml of methanol at 60 °C and 0.92 ml of cc. hydrochloric acid is added dropwise (pH: 2-3). The crystallization starts already at 60 °C, it is cooled to 0 °C in 1 hour and stirred for further 1 hour.
The precipitated product is filtered and washed with 2 x 20 ml of 0 °C methanol and dried to give 4.54 g [yield:63,8% calculated on (-) secondary amine of compound of formula II] of the title compound.

| | |
|---|---|
| Melting point: 234 °C | [α]_{D}²⁰ = -4,4° (c = 0,35 methanol) |
| HPLC purity: >99,85%; | enantiomer pollution:<DL (detection limit) |

### Example 5

1000 tablets are produced by mixing and granulating the following active and inactive ingredients. The obtained tablets are 100 -100 mg of weight

| | |
|---|---|
| R- (-) tamsulosine | 0.4 g |
| Corn starch | 29.6 g |
| Microcrystalline cellulose | 11 g |
| Lactose | 55 g |
| Hydroxy-propyl-cellulose | 3 g |
| Light anhydrous silicic acid | 0.5 g |
| Magnesium stearate | 0.5 g |

## Claims

1. (R,S)-5-[2-(N-benzyl-amino)-propyl]-2-methoxy-benzenesulphonamide of formula II, acid addition salts and enantiomers thereof.

2. R-5-[2-[N-(2-ethoxy-phenoxy)-ethyl]-N-benzyl]-amino]-propyl-2-methoxy-benzenesulphonamide of formula III.

3. Process for the preparation of R-(-)-tamsulosine HCl of formula I, chemical name: R(-)-5-[2-[[2-(2-ethoxy-phenoxy)-ethyl]-amino]-propyl-2-methoxi-benzenesulphonamide hydrochloride **characterized by** reacting (4-methoxy-3-sulphamoyl-phenyl)-acetone and benzylamine in a reductive condensation step, the obtained racemic compound (R,S)-5-[2-(N-benzyl-amino)-propyl]-2-methoxy-benzenesulphonamide of formula II is optically resolved by a chiral acid and then reacted with (2-mesyloxy-ethoxy)-phenetol of formula IV as alkylating agent to R-5-[2-[N-(2-ethoxy-phenoxy)-ethyl]-N-benzyl]-amino]-propyl-2-methoxy-benzene-sulphonamide of formula III, then the benzyl protecting group is removed and the hydrochloride salt is formed.

4. The process according to claim 3, **characterized by** using sodium borohydride as reducing agent in the reductive condensation.

5. The process according to claim 3, **characterized by** using tartaric acid or its derivative as optical resolving agent.

6. The process according to claim 3, **characterized by** using aqueous alcohol as solvent for the optical resolution reaction.

7. The process according to claim 3, **characterized by** that the alkylating reaction is performed by refluxing in an aromatic solvent.

8. The process according to claim 3, **characterized by** using magnesium-oxide as acid naturalize agent in the alkylation reaction.

9. The process according to claim 3, **characterized by** that the removal of the protecting group is carried out by catalytic hydrogenation in the presence of palladium carbon.

## Patentansprüche

1. (R,S)-5-[2-(N-Benzylamino)-Propyl]-2-Methoxybenzolsulphonamid der Formel II, Säurezusatzsalze und die Enantiomere davon.

2. R-5-[2-[N-(2-Ethoxyphenoxy)-Ethyl]-N-Benzyl]-Amino]-Propyl-2-Methoxybenzolsulphonamid der Formel III.

3. Verfahren zur Herstellung von R-(-)-Tamsulosin-HCI der Formel I mit der chemischen Bezeichnung: R(-)-5-[2-[[2-(2-Ethoxyphenoxy)-Ethyl]-Amino]-Propyl-2-Methoxibenzolsulphonamid-Hydrochlorid, **dadurch gekennzeichnet, dass** (4-Methoxy-3-Sulphamoylphenly)-Aceton und Benzylamin in einem reduktiven Kondensationsschritt zur Reaktion gebracht werden, die **dadurch** erhaltene racemische Verbindung (R,S)-5-[2-(N-Benzylamino)-Propyl]-2-Methoxybenzolsulphonamid der Formel II mit einer optisch aktiven Säure aufgelöst und danach mit (2-Mesyloxyethoxy)-Phenol der Formel IV als Alkylierungsmittel zu R-5[2-[N-(2-Ethoxypehnoxy)-Ethyl]-N-Benzyl]-Amino]-Propyl-2-Methoxybenzolsulphonamid der Formel III zur Reaktion gebracht wird und die Benzyl schützende Gruppe sodann entfernt und das Hydrochloridsalz gebildet wird.

4. Verfahren nach Anspruch 3, **dadurch gekennzeichnet, dass** Natriumborhydrid als Reduktionsmittel bei der reduktiven Kondensation eingesetzt wird.

5. Verfahren nach Anspruch 3, **dadurch gekennzeichnet, dass** Weinsäure oder deren Derivat als optisches Auflösungsmittel eingesetzt wird.

6. Verfahren nach Anspruch 3, **dadurch gekennzeichnet, dass** wässriger Alkohol als Lösungsmittel für die optische Auflösungsreaktion eingesetzt wird.

7. Verfahren nach Anspruch 3, **dadurch gekennzeichnet, dass** die Alkylierungsreaktion durch Rückfluss in ein Aromat durchgeführt wird.

8. Verfahren nach Anspruch 3, **dadurch gekennzeichnet, dass** Magnesiumoxid als die Säure naturalisierendes Mittel in der Alkylierungsreaktion eingesetzt wird.

9. Verfahren nach Anspruch 3, **dadurch gekennzeichnet, dass** das Entfernen der Schutzgruppe durch katalytische Hydrierung in Anwesenheit von Palladiumkohlenstoff durchgeführt wird.

## Revendications

1. (R,S)-5-[2-(N-benzyl-amino)-propyl]-2-méthoxy-benzènesulfonamide de formule II, sels d'addition d'acide et énantiomères de celui-ci.

2. R-5-[2-[N-(2-éthoxy-phénoxy)-éthyl]-N-benzyl]-amino]-propyl-2-méthoxy-benzène-sulfonamide de formule III.

3. Procédé de préparation R-(-)tamsulosine HCl de formule I, de nom chimique : chlorhydrate de R(-)-5-[2-[[2-(2-éthoxy-phénoxy)-éthyl]-amino]-propyl-2-méthoxy-benzène-sulfonamide, **caractérisé par** la réaction entre la (4-méthoxy-3-sulfamoyl-phényl)-acétone et la benzylamine dans une étape de condensation réductrice, le composé racémique obtenu (R,S)-5-[2-(N-benzyl-amino)-propyl]-2-méthoxy-benzènesulfonamide de formule II est résolu optiquement par un acide chiral puis réagit avec du (2-mésyloxy-éthoxy)-phénétol de formule IV en tant qu'agent d'alkylation pour donner du R-5-[2-[N-(2-éthoxy-phénoxy)-éthyl]-N-benzyl]-amino]-propyl-2-méthoxy-benzène-sulfonamide de formule III, puis le groupe protecteur benzyle est éliminé et le sel de chlorhydrate est formé.

4. Procédé selon la revendication 3, **caractérisé par** l'utilisation de borohydrure de sodium en tant qu'agent réducteur dans la condensation réductrice.

5. Procédé selon la revendication 3, **caractérisé par** l'utilisation d'acide tartrique ou de son dérivé en tant qu'agent de résolution optique.

6. Procédé selon la revendication 3, **caractérisé par** l'utilisation d'un alcool aqueux en tant que solvant pour la réaction de résolution optique.

7. Procédé selon la revendication 3, **caractérisé en ce que** la réaction d'alkylation est réalisée par mise au reflux dans un solvant aromatique.

8. Procédé selon la revendication 3, **caractérisé par** l'utilisation d'oxyde de magnésium en tant qu'agent de naturalisation d'acide dans la réaction d'alkylation.

9. Procédé selon la revendication 3, **caractérisé en ce que** l'élimination du groupe protecteur est réalisée par hydrogénation catalytique en présence de palladium-carbone.
